# EUROPEAN PATENT APPLICATION

(11) **EP 4 467 137 A1**
(43) Date of publication of application: **27.11.2024**
(21) Application number: 23382486.1
(22) Date of filing: 25.05.2023
(51) Int. Cl.: A61K 31/404, A61K 31/343, A61K 31/381, A61K 31/4355, A61K 31/4365, A61K 31/437, A61K 31/495, A61K 31/4985, A61K 31/5025, A61K 31/53, A61P 25/28

(54) **TREATMENT OF FRONTOTEMPORAL DEMENTIA OR FRONTOTEMPORAL LOBAR DEMENTIA**

(71) Applicant: Universidad Autónoma de Madrid, 28049 Madrid (ES); Consejo Superior De Investigaciones Científicas, 28006 Madrid (ES)
(72) Inventor: GARCÍA LÓPEZ, Manuela, 28049 Madrid (ES); DEL SASTRE LÓPEZ, Eric, 28049 Madrid (ES); MICHALSKA DZIAMA, Patrycja, 28049 Madrid (ES); CUADRADO PASTOR, Antonio, 28049 Madrid (ES); LUENGO MARTÍN, Enrique, 28049 Madrid (ES); FERNÁNDEZ MENDÍVIL, Cristina, 28049 Madrid (ES); RODRÍGUEZ FRANCO, María Isabel, 28006 Madrid (ES); LEÓN MARTÍNEZ, Rafael, 28006 Madrid (ES)
(74) Representative: Hoffmann Eitle

(57) **Abstract**

The present invention refers to the medical field. Particularly, the present invention refers to compounds of *Formula I* or *Formula II* for use in the treatment of frontotemporal dementia or frontotemporal lobar dementia.

## Description

### FIELD OF THE INVENTION

The present invention refers to the medical field. Particularly, the present invention refers to compounds of *Formula I* or *Formula II* for use in the treatment of frontotemporal dementia or frontotemporal lobar dementia.

### STATE OF THE ART

Frontotemporal dementia (FTD) is the second most common early-onset dementia and is characterised clinically by progressive behavioural changes and frontal executive deficits and/or selective language difficulties. The clinical spectrum of FTD encompasses distinct canonical syndromes: the behavioural variant of FTD (bvFTD) and the language variants, semantic dementia (SD) and progressive non-fluent aphasia (PNFA). The syndrome is also called frontotemporal lobar degeneration (FTLD). However, FTLD refers to the pathological - rather than clinical - syndrome characterized by atrophy of prefrontal and anterior temporal cortices.

FTD occurs in 5-15% of patients with dementia and it is the third most common degenerative dementia. FTD occurs with equal frequency in both sexes. The age of onset is usually between 45 and 65 years though it may range anywhere from 21 to 81 years. The usual course is one of progressive clinicopathological deterioration with mortality within 6-8 years. Unlike Alzheimer's disease, this condition has a strong genetic basis and family history is seen in 40-50% of cases. FTD is a genetically complex disorder inherited as an autosomal dominant trait with a high penetrance in most cases. The most frequently mutated genes are *GRN* (progranulin) and *MAPT* (microtubule-associated protein tau).

Until recently two major pathological types of FTLD were described, those with tau-positive pathology (FTLD-tau) and those with tau-negative, ubiquitin-positive pathology (FTLD-U). However, it has been shown that FTLD-U consists of three separate groups: those with TDP-43-positive inclusions (FTLD-TDP), those with FUS-positive inclusions (FTLD-FUS) and a minority of cases which are both TDP-43 and FUS-negative (now called FTLD-UPS). Each of these major pathological types also has several subtypes **(Table 1).**

Thus, one of the most common causes of FTLD is the abnormal deposition of tau proteins, which are a group of six highly soluble protein isoforms produced by alternative splicing from the *MAPT* gene.

**Table 1:**

| Summary of the classes and subtypes of FTD/FTLD and the genes that are mutated in each of these conditions, according to Mackenzie et al. (2010) [Mackenzie, Ian R A et al. "Nomenclature and nosology for neuropathologic subtypes of frontotemporal lobar degeneration: an update." Acta neuropathologica vol. 119,1 (2010): 1-4. doi:10.1007/s00401-009-0612-2]. | | |
|---|---|---|
| **Major molecular class** | **Recognised subtypes** | **Associated gene/locus** |
| FTLD-tau | Pick's disease (PiD) | *MAPT* |
| | Corticobasal degeneration (CBD) | |
| | Progressive supranuclear palsy (PSP) | |
| | Argyrophilic grain disease (AGD) | |
| | Multiple system tauopathy with dementia (MSTD) | |
| | Neurofibrillary tangle predominant dementia (NFT-dementia) | |
| | White matter tauopathy with globular glial inclusions (WMT-GGI) | |
| | Unclassifiable | |
| FTLD-TDP | Types 1-4 | *GRN* |
| | Unclassifiable | *VCP* |
| | | Genetic locus on chromosome 9p |
| | | *TARDBP* |
| FTLD-UPS | Frontotemporal dementia linked to chromosome 3 (FTD-3) | *CHMP2B* |
| FTLD-FUS | Atypical frontotemporal lobar degeneration with ubiquitinated inclusions (aFTLD-U) | *FUS* |
| | Neuronal intermediate filament inclusion disease (NIFID) | |
| | Basophilic inclusion body disease (BIBD) | |
| FTLD-ni (no inclusions) | | |

There is currently no cure for FTD and FTDL. Thus, there is an unmet medical need of finding effective treatments against FTD and FTDL. The present invention is focused on solving this problem and an effective therapeutical compound is herein provided.

### DESCRIPTION OF THE INVENTION

### Brief description of the invention

The present invention refers to compounds of *Formula I* or *Formula II* for use in the treatment of frontotemporal dementia or frontotemporal lobar dementia.

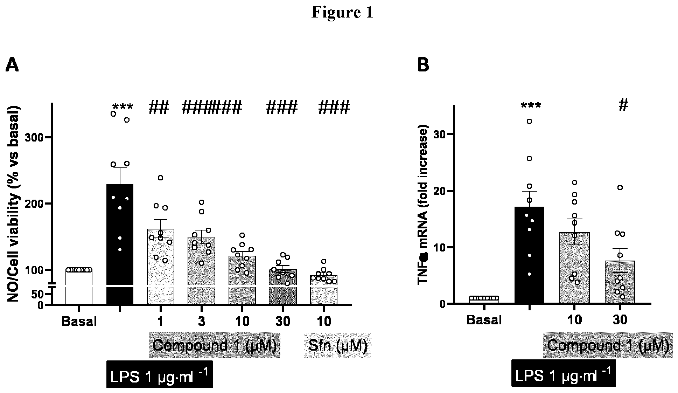

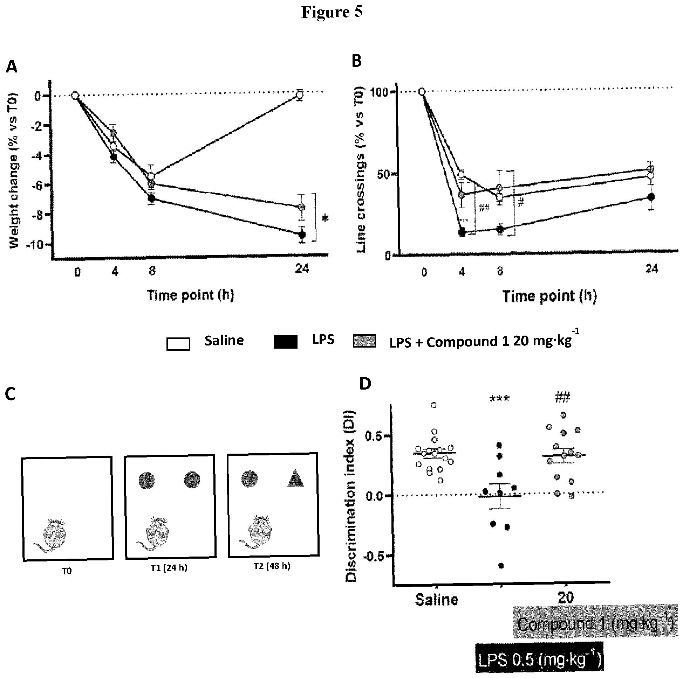

The present invention refers to compounds of *Formula I* or *Formula II* that have anti-neuroinflammatory properties *in vitro* **(Table 2).** Based on the results obtained from an *in vitro* characterization of the molecules of **Table 2,** Compound 1 of **Table 2,** showing the highest anti-inflammatory properties, was selected for further *in vitro* and *in vivo* studies. Compound I showed anti-inflammatory properties in neural cells and mouse brain subjected to the toxic action of the pro-inflammatory stimulus lipopolysaccharide. More precisely, Compound 1:
- Significantly reduced the expression of inflammatory cytokines in microglial cells *in vitro* **(****Figure 1****).**
- Significantly reduced the expression of inflammatory cytokines **(****Figure 2** and **Figure 3****),** as well as the presence of microglial cells - which constitute the immune cells of the brain - in brain **(****Figure 4****).**
- Significantly reduced LPS-induced "sickeness behaviour" in mice, which involves body weight loss, reduction of the locomotor activity and a cognitive decline **(****Figure 5****).** Of note, LPS-induced "sickening behaviour" is a well-known model of neuroinflammation.

Since neuroinflammation plays a major role in FTD [Bright, Fiona et al. "Neuroinflammation in frontotemporal dementia." Nature reviews. Neurology vol. 15,9 (2019): 540-555. doi:10.1038/s41582-019-0231-z], it is expected that the identified compounds could have a therapeutic effect in the development of FTD. Using Compound 1 as a proof-of concept, the inventors assessed the therapeutic effect of these compounds in FTD both *in vitro* and *in vivo,* in the context of expression of an exogenous human tau gene with a P301L mutation. Importantly, the P301L tau mutation has been associated with familial forms of frontotemporal dementia [Nasreddine, Z S et al. "From genotype to phenotype: a clinical pathological, and biochemical investigation of frontotemporal dementia and parkinsonism (FTDP-17) caused by the P301L tau mutation." Annals of neurology vol. 45,6 (1999): 704-15. doi:10.1002/1531-8249(199906)45:6<704:aid-ana4>3.0.co;2-x; Mirra, S S et al. "Tau pathology in a family with dementia and a P301L mutation in tau." Journal of neuropathology and experimental neurology vol. 58,4 (1999): 335-45. doi:10.1097/00005072-199904000-00004] and the mouse model carrying that mutation recapitulates the progressive development of glial and neurofibrillary tangles, and associated cerebral atrophy observed in patients suffering from FTD [Kent, Brianne A et al. "Longitudinal evaluation of Tau-P301L transgenic mice reveals no cognitive impairments at 17 months of age." Brain and behavior vol. 8,1 e00896. 18 Dec. 2017, doi:10.1002/brb3.896]. Thus, mice carrying the P301L tau mutation were used as an *in vivo* model of FTD.

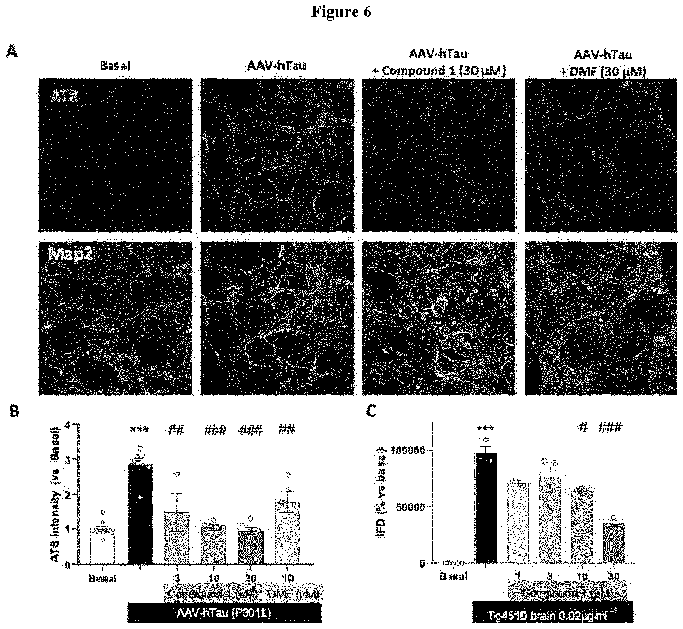

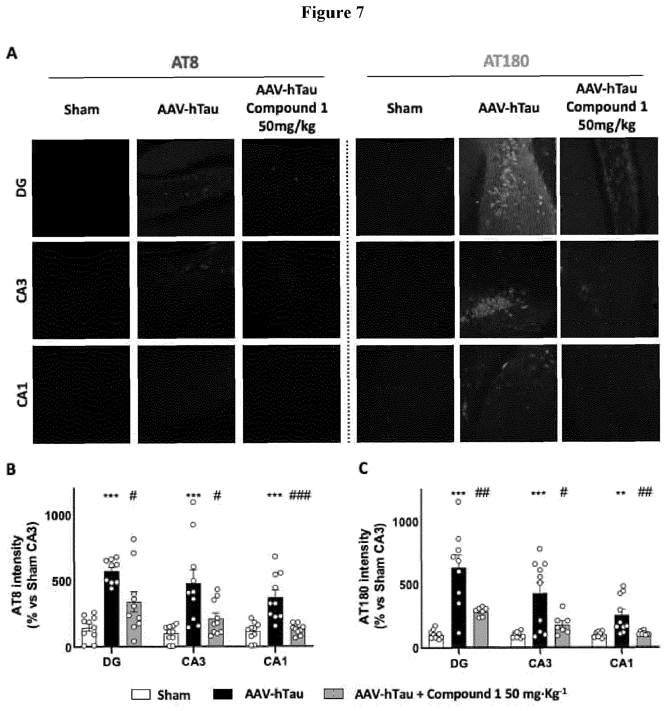

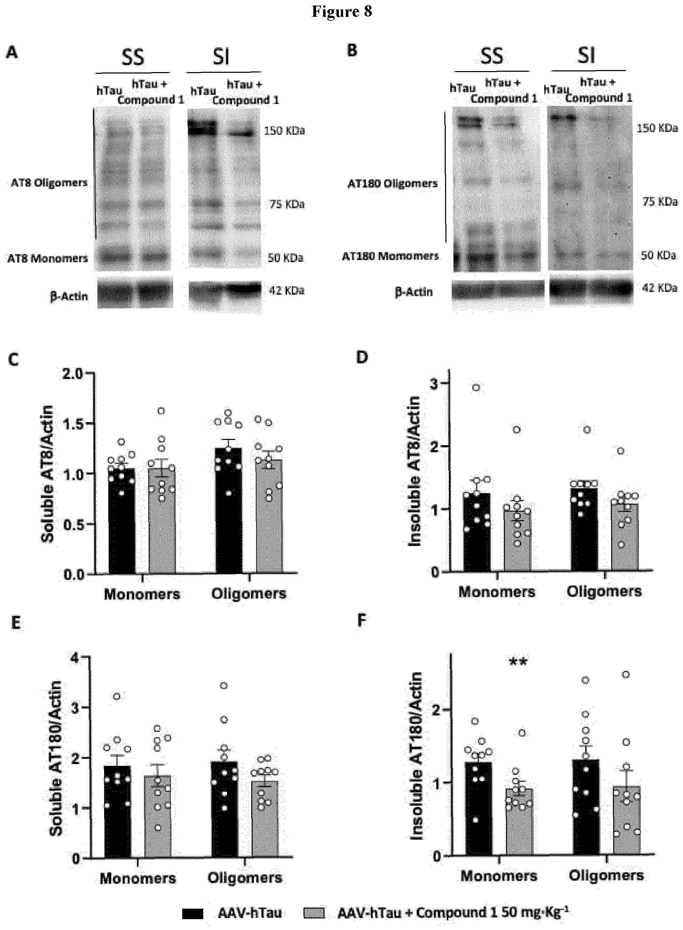

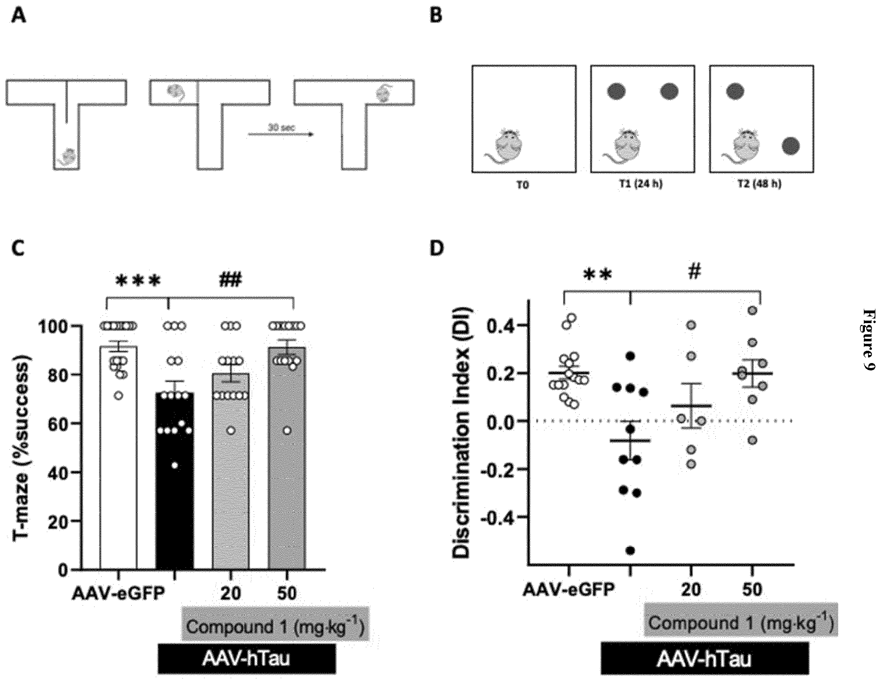

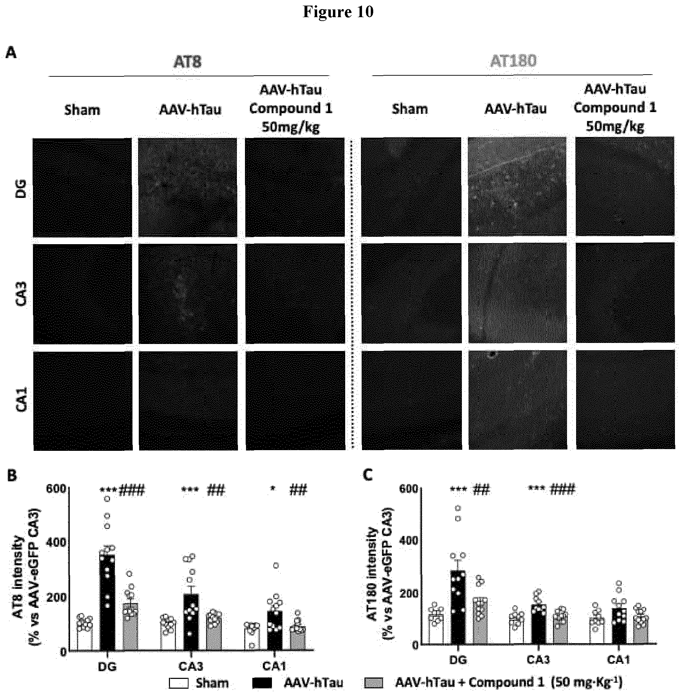

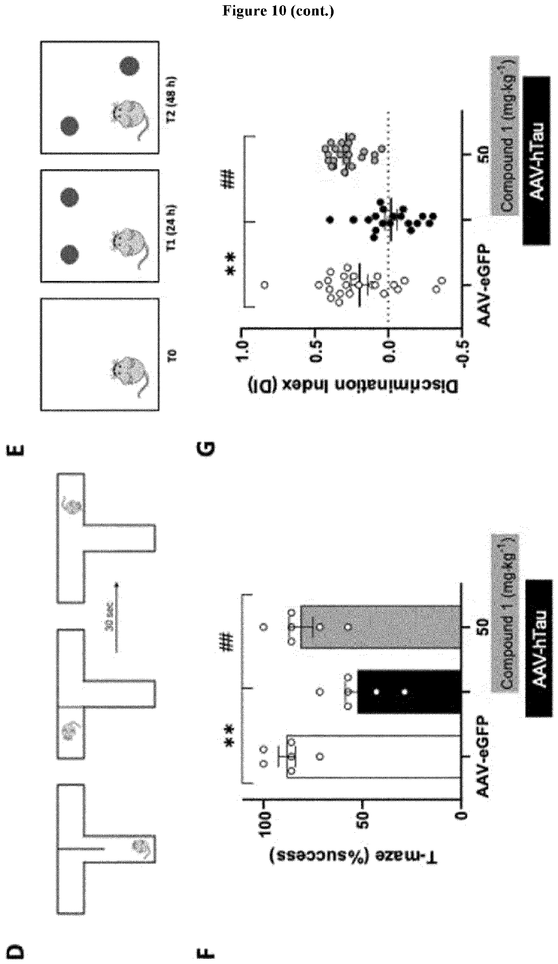

The most important conclusions from these analyses are:
- Compound 1 reduces levels of pathological hyperphosphorylated tau in primary cultures of neurons treated with adeno-associated particles expressing human tau protein with P301L mutation **(****Figure 6****).**
- Upon FTD induction in mice by bihippocampal injection of adeno-associated particles expressing the human P301L mutant tau protein:
   ∘ Oral administration of a single daily dose of 50 mg/kg Compound 1 for 35 days post-induction of FTD decreases hyperphosphorylated tau accumulation in 3-month-old mice **(****Figure 7****).**
   ∘ Oral administration of a single daily dose of 50 mg/kg Compound 1 for 35 days post-induction of FTD decreases the accumulation of insoluble phosphorylated tau in 3-month-old mice **(****Figure 8****).**
   ∘ Oral administration of a single daily dose of 20 mg/kg or 50 mg/kg of Compound 1 for 35 days postinduction of FTD, dose-dependently prevents cognitive deficits, as assessed in two hippocampal-dependent memory tests in 3-month-old adult mice **(****Figure 9****).**
   ∘ Oral administration of a single daily dose of 50 mg/kg Compound 1 for 35 days post-induction of FTD decreases the accumulation of hyperphosphorylated tau in 18-month-old aged mice **(****Figure 10A-C).**
   o Oral administration of a single daily dose of 50 mg/kg Compound 1 for 35 days post-induction of FTD prevents cognitive deficits in 18-month-old aged mice **(****Figure 10D-G).**

Thus, the first embodiment of the present invention refers to a compound characterized by comprising the *Formula I,* or salts or derivatives thereof, 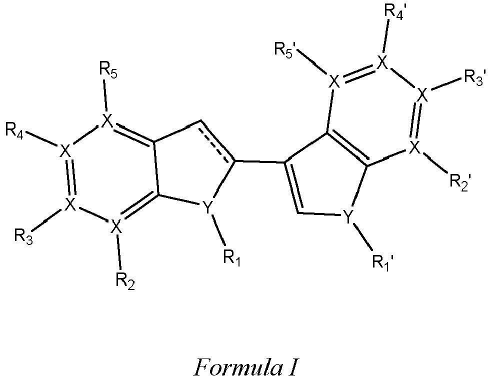 wherein,
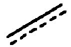 represents a double or single bond,
**X** is either C or N,
**Y** is N, O or S,
**R₁** and **R₁'** are independently absent, H, C₁₋₄ alkyl, or an acyl group CO**R₆;** wherein
**R₆** is H or C₁₋₄ alkyl; and wherein the C₁₋₄ alkyl comprises cycloalkyl, branched alkyl, alkenyl or alkynyl, optionally substituted with 1-3 halogen atoms,
**R₂**, **R₃**, **R₄, R₅, R₂' R₃', R₄'** and **R₅'** are independently absent, H, carbonitrile, C₁₋₄ alkyl, a halogen, aryl, heteroaryl, an alkoxy group O**R₇**, or an amino group N**R**₈**R**₉;
wherein **R**₇, **R**₈ and **R**₉ are H or C₁₋₄ alkyl; and wherein C₁₋₄ alkyl comprises cycloalkyl, branched alkyl, alkenyl or alkynyl, optionally substituted with 1-3 halogen atoms;
for use in the treatment of frontotemporal dementia or frontotemporal lobar dementia.

Kindly note that when X is N; R₂ R₃, R₄, and R₅ and additionally R₂' R₃', R₄', and R₅' are absent. When Y is O or S; R₁ and R₁' are absent.

In a preferred embodiment, the present invention refers to a compound characterized by comprising the *Formula II,* or salts or derivatives thereof, 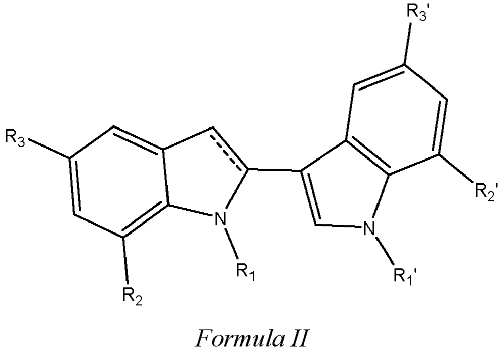 wherein,
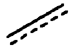 represents a double or single bond,
**R₁** and **R₁'** are independently H or C₁₋₄ alkyl, wherein the C₁₋₄ alkyl comprises cycloalkyl, branched alkyl, alkenyl or alkynyl, optionally substituted with 1-3 halogen atoms,
**R₂** and **R₂'** are independently H or an alkoxy group O**R₇**, where **R₇** is H or C₁₋₄ alkyl, wherein the C₁₋₄ alkyl comprises cycloalkyl, branched alkyl, alkenyl or alkynyl, optionally substituted with 1-3 halogen atoms,
**R₃** and **R₃'** are independently H or an alkoxy group O**R₇**, where **R₇** is H, C₁₋₄ alkyl, wherein the C₁₋₄ alkyl comprises cycloalkyl, branched alkyl, alkenyl or alkynyl optionally substituted with 1-3 halogen atoms, a halogen atom or C₁₋₄ alkyl comprising cycloalkyl, optionally substituted with 1-3 halogen atoms,
for use in the treatment of frontotemporal dementia or frontotemporal lobar dementia.

In a preferred embodiment, the compounds of the invention are characterized in that, when **R₁** and **R₁'** are both H or prop-2-ynyl, **R₂** and **R₂'** are both H or methoxy and/or **R₃** and **R₃'** are both H, methoxy, chloro or trifluoromethyl.

In a preferred embodiment, the compounds of the invention are characterized in that, when **R₁** and **R₁'** are both H, **R₂** and **R₂'** are both H or methoxy and **R₃** and **R₃'** are both H, methoxy, chloro or trifluoromethyl.

In a preferred embodiment, the compounds of the invention are characterized in that **R₂** and **R₂'** are both H or methoxy and **R₃** and **R₃'** are both H.

In a preferred embodiment, the compounds of the invention are characterized in that **R₃** and **R₃'** are both H, methoxy, chloro or trifluoromethyl and **R₂** and **R₂'** are both H.

In a preferred embodiment, the compounds of the invention are characterized in that **R₁** and **R₁'** are both prop-2-ynyl, **R₃** and **R₃'** are both methoxy and **R₂** and **R₂'** are both H.

In a preferred embodiment, the compounds of the invention are selected from the group comprising:
5-Methoxy-3-(5-methoxyindolin-2-yl)-1*H*-indole **(1)**
5,5'-Dimethoxy-1*H*, 1'*H*-2,3'-biindole **(2)**
3-(Indolin-2-yl)-1H-indole **(3)**
5-Chloro-3-(5-chloroindolin-2-yl)-1*H*-indole **(4)**
5,5'-Dichloro-1*H*,1'*H*-2,3'-biindole **(5)**
7-Methoxy-3-(7-methoxyindolin-2-yl)-1*H*-indole **(6)**
5-(Trifluoromethyl)-3-(5-(trifluoromethyl)indolin-2-yl)-1*H*-indole **(7)**
5-Methoxy-3-(5-methoxy-1-(prop-2-yn-1-yl)indolin-2-yl)-1-(prop-2-yn-1-yl)-1*H*-indole **(8)**

In a preferred embodiment, the compound of the invention is:
5-Methoxy-3-(5-methoxyindolin-2-yl)-1*H*-indole **(1)**

In a preferred embodiment, the frontotemporal dementia or frontotemporal lobar dementia is characterized by the presence of mutations in the MAPT gene.

In a preferred embodiment, the frontotemporal dementia or frontotemporal lobar dementia is selected from the list comprising: Pick's disease, corticobasal degeneration, progressive supranuclear palsy, argyrophilic grain disease, multiple system tauopathy with dementia, neurofibrillary tangle predominant dementia and/or white matter tauopathy with globular glial inclusions.

In a preferred embodiment, the frontotemporal dementia or frontotemporal lobar dementia is characterized by the presence of mutation P301L in the MAPT gene.

In a preferred embodiment, the compound of the invention is administered *via* parenteral or enteral route, preferably via sublingual, buccal, oral and rectal routes.

In a preferred embodiment, the compound of the invention is administered *via* enteral or parenteral route at a concentration ranging from 20 to 100 mg/kg.

The second embodiment of the present invention refers to a pharmaceutical composition comprising the *Formula I or Formula II,* or salts or derivatives thereof, and optionally pharmaceutically acceptable excipients and/or carriers, for use in the treatment of frontotemporal dementia or frontotemporal lobar dementia.

Alternatively, the present invention also refers to a method for treating frontotemporal dementia or frontotemporal lobar dementia which comprises the administration of a therapeutically effective dose or amount of any of the compound of **Table 2** or compositions comprising thereof.

In the context of the present invention the following terms are defined:
- The term "comprising" means including, but it is not limited to, whatever follows the word "comprising". Thus, use of the term "comprising" indicates that the listed elements are required or mandatory, but that other elements are optional and may or may not be present.
- The term "consisting of" means including, and it is limited to, whatever follows the phrase "consisting of". Thus, the phrase "consisting of" indicates that the listed elements are required or mandatory, and that no other elements may be present.
- "Pharmaceutically acceptable excipient or carrier" refers to an excipient that may optionally be included with the pharmaceutical composition of the invention and that causes no significant adverse toxicological effects to the patient.
- By "therapeutically effective dose or amount" of the pharmaceutical composition of the invention is intended an amount that, when administered as described herein, brings about a positive therapeutic response in a subject suffering from frontotemporal dementia or frontotemporal lobar dementia. The exact amount required will vary from subject to subject, depending on the age, and general condition of the subject, the severity of the condition being treated, mode of administration, and the like. An appropriate "effective" amount in any individual case may be determined by one of ordinary skill in the art using routine experimentation, based upon the information provided herein.

### Description of the figures

**Figure 1****. Compound 1 reduces the inflammatory response against LPS *in vitro.* (A)** Nitric oxide (NO) production in BV2 cells detected by DAF 24 h after LPS exposure and normalized by cell viability measured by MTT (n = 9). **(B)** qRT-PCR of TNF_{α} (n = 9) in BV2 cells after 24 h LPS exposure. Sulforaphane (Sfn) was used as a positive control. Significant differences were considered when: *** p<0.001 shows significant differences compared to the basal condition and # p< 0.05 ## p< 0.01, ### p< 0.001 compared to the LPS condition. h: hours.

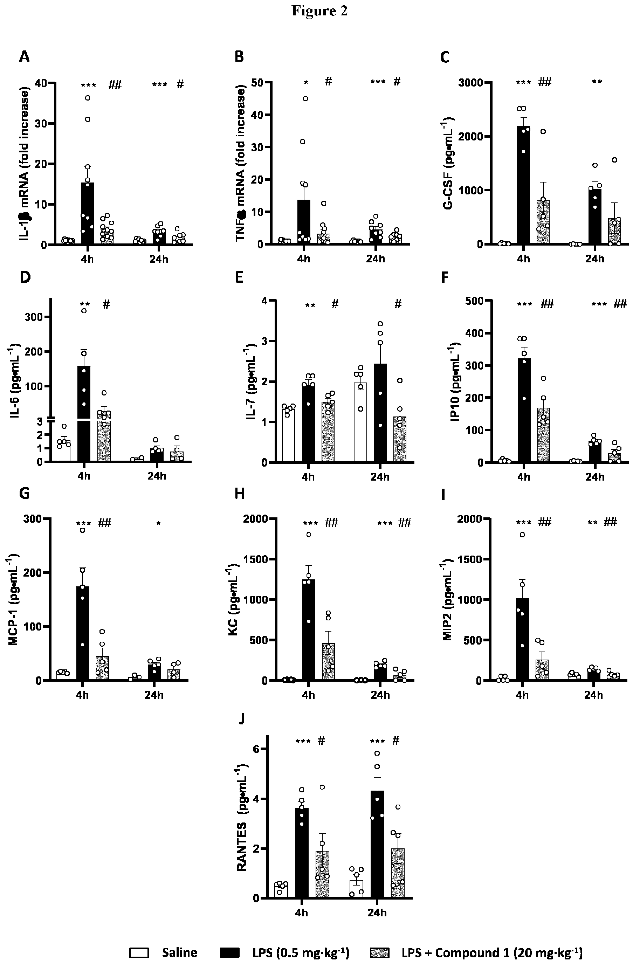

**Figure 2****. Compound 1 decreases the inflammatory process 4 and 24 hours after LPS administration *in vivo.*** Compound 1 (20 mg·kg⁻¹) daily was injected i.p. four days before the i.p. LPS (0.5 mg·kg⁻¹) administration. Thereafter, proinflammatory cytokines were measured by qRT-PCR at 4 h (Ilβ (n = 10, except LPS n = 9) and 24 h (n = 8) (A) and TNFα at 4 h (n = 10, except LPS n = 9) and 24 h (n = 7-8) **(B).** Multiplex assay of cytokines was also evaluated 4 h and 24 h after the proinflammatory stimuli: G-CSF (n = 5) **(C),** IL-6 (n = 5) **(D),** IL-7 (n = 5) **(E),** IP10 (n = 5) **(F),** KC (4 h n = 5; 24 h: n = 5 except saline n = 3) **(G),** MCP-1 (n = 5) **(H),** MIP2 (n = 5) **(I)** and RANTES (n = 5) **(J).** Data represent mean ± SEM. * shows differences compared to the basal condition and # to the LPS condition. h: hours.

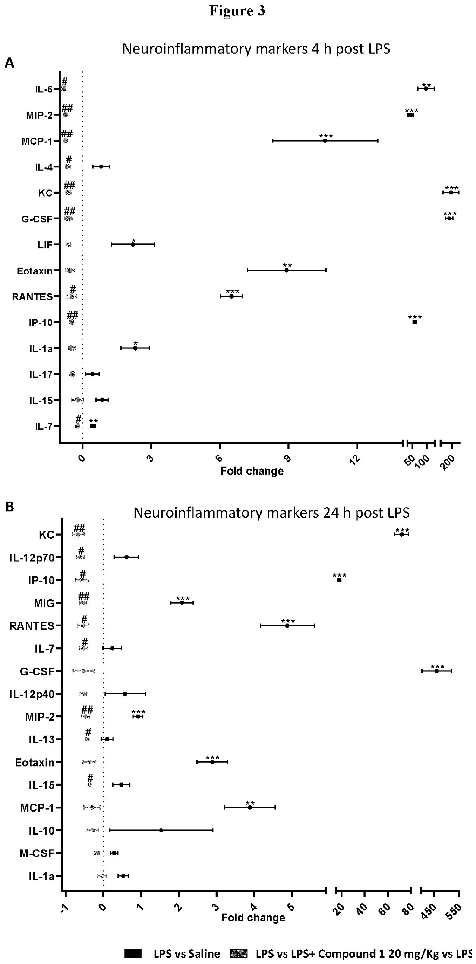

**Figure 3****. Compound 1 ameliorates the resolution of the inflammatory process triggered by LPS.** Multiplex assay of cytokines 4 h **(A)** and 24 h **(B)** after the proinflammatory stimuli (n = 5). Data represent mean ± SEM. * shows differences compared to the basal condition and # to the LPS condition. h: hours.

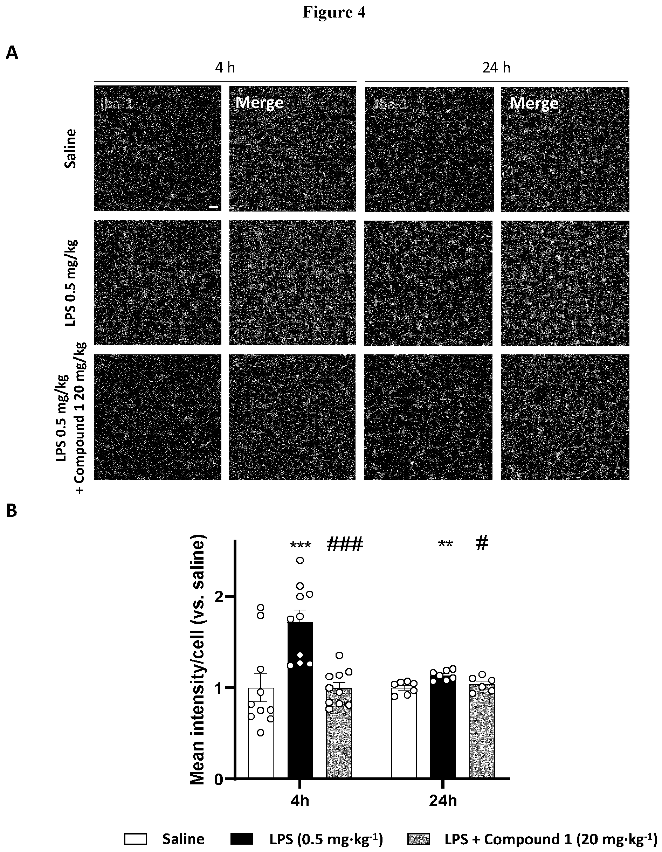

**Figure 4****. Compound 1 reverts brain microgliosis triggered by LPS.** Representative images of brain microglia 4 h and 24 h after the proinflammatory stimuli **(A).** Mean intensity of IBA-1 per cell 4 h (n = 10) and 24 h (n = 7, except n = 6 LPS + Compound 1) after LPS injection **(B).** Data represent mean ± SEM. * shows differences compared to the basal condition and # to the LPS condition. h: hours.
**Figure 5****. Sub-chronical Compound 1 administration improves behavioural parameters in LPS treated mice.** Compound 1 (20 mg·kg⁻¹) was injected once a day via i.p. four days before the i.p. LPS (0.5 mg·kg⁻¹) administration, thereafter behavioural tests were conducted. **(A)** Mice weight change (T0 and T24: saline n = 17, LPS n = 17, LPS + Compound 1 n = 19, T4 and T8 saline n = 8, LPS n = 7, LPS + Compound 1 n = 8). **(B)** Locomotion activity measured by line crossings of an imaginary grid. **(C)** Scheme of NOR test. **(D)** Discrimination index (DI) of the mice studied: saline (n = 16), LPS (n = 9) and LPS + Compound 1 (n = 13). Data represent mean ± SEM. * shows differences compared to the basal condition and # to the LPS condition. h: hours.
**Figure 6****. Compound 1 reduces hyperphosphorylated tau species and tau seeding *in vitro.*** Compound 1 reduces hyperphosphorylated tau levels (AT8) in primary neuronal cultures of mice after 8 days of AVV-hTauP301L treatment. Dimethyl fumarate was used as a positive control (DMF). **(A)** Representative images, AT8 is shown in green and Map2, marker of neurons, is shown in white. **(B)** AT8 intensity quantification (n: Basal and AAV-hTau = 8, AAV-hTau + compound 1 at 3 mM = 3, AAV-hTau + Compound 1 at 10 mM = 6, AAV-hTau + Compound 1 at 10 mM = 6, and AAV-hTau + DMF 10 mM = 5). * Shows differences compared to the basal condition and # compared to the AAV-hTau condition. Compound 1 reduced tau seeding in HEK-293 tau FRET biosensor measured by flow cytometer **(C).** Tg4510 mouse brain homogenate was used as aggregant stimuli. Integrated FRET density (IFD) was calculated multiplying percentage of cells containing aggregates and mean fluorescence intensity and normalized to basal condition. * Shows differences compared to the basal condition and # compared to the Tg4510 treated condition. Data represent mean ± SEM.
**Figure 7****. Compound 1 reduces hyperphosphorylated tau species in adult mice with hTauP301L tauopathy after 35 days oral administration (50 mg/kg/day). (A).** Representative images of hyperphosphorylated tau intensity measured by AT8 and AT180 antibodies in three different regions of the hippocampus (GD, CA3 and CA1) (n = 11-12). **(B)** Quantification AT8 intensity, normalized to CA3 intensity of Sham animals. **(C)** Quantification of AT180 intensity, normalized to CA3 intensity of Sham animals (n = 9-12). Data represent mean ± SEM. * Shows differences compared to the Sham condition and # compared to AAV-hTau condition.
**Figure 8****. Compound 1 reduces insoluble forms of hyperphosphorylated tau species in adult mice with hTauP301L tauopathy after 35 days oral administration (50 mg/kg/day). (A).** Representative images of WB against AT8 and **(B)** AT180 antibodies of sarkosyl solule and insoluble protein species. Quantification of sarkosyl soluble (n = 10) **(C)** and insoluble species **(D)** using AT8 antibody (n = 10). Quantification of sarkosyl soluble (n = 10) **(E)** and insoluble species **(F)** using AT180 antibody (n = 10). Data represent mean ± SEM. * shows differences compared to the AAV-hTau condition.
**Figure 9****. Compound 1 prevents cognitive decline in spatial memory behavioural paradigms in adult mice with hTauP301L tauopathy after 35 days oral administration (20 mg/kg or 50 mg/kg/day). (A)** Representation of T-maze alternation test and **(B)** Object Location test (OLT). **(C)** Average percentage of success in seven independent attempts (n = 14-19). **(D)** Discrimination index in the OLT assay (AAV-eGFP, n = 15; AAV-hTau, n = 10; AAV-hTau + Compound 1 at 20 mg/kg, n = 5; AAV-hTau + Compound 1 at 50 mg/kg, n = 8). Data represent mean ± SEM. * shows differences compared to the Sham condition and # compared to AAV-hTau condition.
**Figure 10****. Compound 1 reduces hyperphosphorylated tau species and improves cognitive behaviour in aged (16-18 months old) mice with hTauP301L tauopathy after 35 days oral administration (50 mg/kg/day). (A).** Representative images of hyperphosphorylated tau intensity measured by AT8 and AT180 antibodies in three different regions of the hippocampus (GD, CA3 and CA1) (n = 12). **(B)** Quantification AT8 intensity, normalized to CA3 intensity of Sham animals. **(C)** Quantification of AT180 intensity, normalized to CA3 intensity of Sham animals. **(D)** Experimental protocol of T-maze alternation test and **(E)** Object Location test (OLT). **(F)** Average of the percentage of success in seven independent attempts (n = 6). **(G)** Discrimination index in the OLT assay (AAV-eGFP n = 23, AAV-hTau n=19 AAV-hTau + Compound 1 at 50 mg/kg (n = 22). Data represent mean ± SEM. * shows differences compared to the Sham condition and # compared to AAV-hTau condition.

### Detailed description of the invention

The present invention is illustrated by means of the Examples set below without the intention of limiting its scope of protection.

### Example 1. Material and Methods

### Example 1.1. Synthesis of compounds

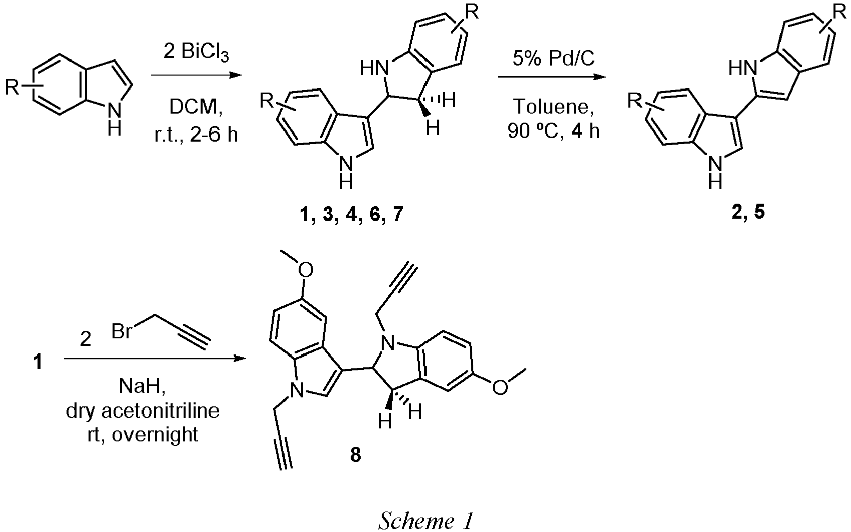

Synthetic methods are common and obvious to those skilled in the art.

5-Methoxy-3-(5-methoxyindolin-2-yl)-1*H*-indole **(1).** White solid, 43% yield, mp 170-171 °C. ¹H NMR (400 MHz, DMSO) δ 10.70 - 10.65 (m, 1H), 7.23 (d, *J=* 8.7 Hz, 1H), 7.20 (d, *J* = 2.5 Hz, 1H), 6.91 (d, *J* = 2.4 Hz, 1H), 6.75 (d, *J* = 2.5 Hz, 1H), 6.71 (dd, *J* = 8.7, 2.5 Hz, 1H), 6.56 (dd, *J* = 8.4, 2.6 Hz, 1H), 6.49 (d, *J* = 8.3 Hz, 1H), 5.46 (d, *J* = 2.7 Hz, 1H), 5.04 (td, *J =* 8.5, 2.5 Hz, 1H), 3.66 (s, 3H), 3.65 (s, 3H), 3.34 (m, 1H, DMSO-d6), 2.95 (dd, *J* = 15.6, 8.1 Hz, 1H). ¹³C NMR (101 MHz, DMSO) δ 153.3, 152.5, 146.6, 132.4, 130.4, 126.4, 123.1, 118.7, 112.5, 112.5, 111.7, 111.6, 108.9, 101.7, 56.7, 55.9, 55.7, 38.0. HPLC-MS (10-95, gt: 10 min) t_{R}: 4.40 min, *m*/*z* =295 [M+H]⁺, purity > 99%.

5,5'-Dimethoxy-1*H*,1'*H*-2,3'-biindole **(2).** White solid, 62% yield, mp 192-193 °C. ¹H NMR (500 MHz, acetone-d₆) δ 10.62 (bs, 1H), 10.47 (bs, 1H), 7.80 (d, *J=* 1.6 Hz, 1H), 7.42 (d, *J=* 1.2 Hz, 1H), 7.36 (d, *J* = 5.6 Hz, 1H), 7.19 (d, *J* = 5.6 Hz, 1H), 7.03 (d, *J* = 1.6 Hz, 1H), 6.77 (dd, *J* = 16.0, 5.6 Hz, 1H), 6.74 (dd, *J* = 16.0, 5.6 Hz, 1H), 6.67 (dd, *J* = 16.0, 5.6 Hz, 1H), 6.68 (m, 2H), 3.86 (s, 3H), 3.76 (s, 3H). ¹³C NMR (125 MHz, acetone-d₆) δ 156.05, 154.55, 142.37, 133.77, 132.28, 128.46, 127.56, 123.11, 114.86, 112.93, 112.68, 112.30, 112.07, 106.06, 104.13, 100.71, 55.55, 55.34. HPLC-MS (10-95, gt: 10 min) t_{R}: 3.18 min, *m*/*z* =293 [M+H]⁺, purity = 99%.

3-(Indolin-2-yl)-1*H*-indole **(3).** White solid, 58% yield, mp 105-106 °C. ¹H NMR (500 MHz, acetone-d₆) δ 7.72 - 7.64 (m, 2H), 7.41 (dd, *J =* 7.5, 1.4 Hz, 1H), 7.23 (td, *J* = 7.5, 1.5 Hz, 1H), 7.17 - 7.09 (m, 2H), 7.02 (ddt, *J* = 7.5, 2.0, 1.0 Hz, 1H), 6.98 (td, *J* = 7.5, 2.0 Hz, 1H), 6.78 (td, *J* = 7.5, 2.0 Hz, 1H), 6.60 (dd, *J* = 7.5, 2.0 Hz, 1H), 5.28 (t, *J* = 5.6 Hz, 1H), 4.42 (s, 1H), 3.38 (ddd, *J* = 18.3, 5.6, 1.0 Hz, 1H), 3.29 (ddd, *J* = 18.3, 5.6, 1.0 Hz, 1H). ¹³C NMR (126 MHz, acetone-d₆) δ 150.92, 134.87, 128.43, 125.67, 123.78, 123.52, 121.34, 113.25, 111.59, 110.51, 57.53, 35.75. HPLC-MS (10-95, gt: 10 min) t_{R}: 4.35 min, *m*/*z* =235 [M+H]⁺, purity = 98%.

5-Chloro-3-(5-chloroindolin-2-yl)-1*H*-indole **(4).** White solid, 64% yield, mp 122-123 °C. ¹H NMR (500 MHz, acetone-d₆) δ 9.05 (bs, 1H), 8.10 (bs, 1H), 7.64 (d, *J* = 2.0 Hz, 1H), 7.37 (d, *J* = 8.4 Hz, 1H), 7.15-7.05 (m, 3H), 6.89 (dd, *J=* 8.4, 2.4 Hz, 1H), 6.76 (d, *J=* 8.4 Hz, 1H), 5.26 (t, *J* = 8.8 Hz, 1H), 3.52 (dd, *J* = 15.6, 9.2 Hz, 1H), 3.46 (dd, *J* =15.6, 8.8 Hz, 1H). ¹³C NMR (126 MHz, acetone-d₆) δ 150.15, 134.57, 131.82, 129.48, 128.81, 127.54, 126.06, 125.58, 123.12, 122.24, 120.87, 119.83, 113.59, 110.05, 57.82, 39.23. HPLC-MS (10-95, gt: 10 min) t_{R}: 4.83 min, *m*/*z* =303 [MH]⁺, 305 [MH+2]⁺, 307 [MH+4]⁺, purity = 98%.

5,5'-Dichloro-1*H*,1'*H*-2,3'-biindole **(5).** White solid, 78% yield, mp 237-238 °C. ¹H NMR (500 MHz, acetone-*d*₆) δ 10.80 (s, 1H), 10.63 (s, 1H), 8.03 (dd, *J =* 2.1, 0.7 Hz, 1H), 7.97 - 7.84 (m, 1H), 7.58 (dd, *J* = 1.7, 1.1 Hz, 1H), 7.54 (dd, *J* = 8.6, 0.6 Hz, 1H), 7.38 (dt, *J =* 8.5, 0.7 Hz, 1H), 7.29 - 7.12 (m, 1H), 7.10 - 6.99 (m, 1H), 6.83 (dd, *J =* 2.2, 0.9 Hz, 1H). ¹³C NMR (126 MHz, acetone-*d*₆) δ 135.54, 135.21, 135.03, 130.93, 126.23, 125.50, 124.72, 124.41, 122.27, 120.64, 118.93, 118.65, 113.32, 113.27, 111.73, 97.66. HPLC-MS (10-95, gt: 10 min) t_{R}: 3.17 min, *m*/*z* =301 [MH]⁺, 303 [MH+2]⁺, 305 [MH+4]⁺, purity = 97%.

7-Methoxy-3-(7-methoxyindolin-2-yl)-1*H*-indole **(6).** White solid, 45% yield, mp 168-169 °C. ¹H NMR (300 MHz, acetone-*d*6) δ 10.05 (bs, 1H), 7.24 (d, *J* = 2.4 Hz, 1H), 7.17 (d, *J* = 8.0 Hz, 1H), 6.90 (t, *J* = 8.0 Hz, 1H), 6.74 (d, *J* = 7.8 Hz, 1H), 6.72 (d, *J* = 8.0 Hz, 1H), 6.65 (d, *J* = 7.8 Hz, 1H), 6.62 (t, *J* = 7.8 Hz 1H), 5.23 (t, *J* = 9.0 Hz, 1H), 4.63 (bs, 1H), 3.92 (s, 3H), 3.79 (s, 3H), 3.44 (dd, *J =* 15.3, 9.0 Hz, 1H), 3.10 (dd, *J* = 15.3, 9.0 Hz, 1H). ¹³C NMR (126 MHz, acetone-*d*6) δ 206.35, 147.42, 145.84, 141.57, 130.28, 128.47, 128.34, 122.14, 120.21, 120.11, 119.24, 117.90, 113.06, 110.47, 102.47, 57.93, 55.63, 55.56, 39.07. HPLC-MS (15-95, gt: 10 min) t_{R}: 5.95 min, *m*/*z* = 295 [MH]⁺, purity = 98%.

5-(Trifluoromethyl)-3-(5-(trifluoromethyl)indolin-2-yl)-1*H*-indole (7). White solid, 60% yield. ¹H NMR (500 MHz, acetone-*d*₆) δ 10.56 (s, 1H), 7.93 (dt, *J* = 1.8, 0.9 Hz, 1H), 7.61 (dt, *J* = 8.6, 0.8 Hz, 1H), 7.53 (dd, *J* = 2.5, 0.6 Hz, 1H), 7.41 (dd, *J* = 8.6, 1.8 Hz, 1H), 7.39 - 7.29 (m, 2H), 6.71 (d, *J =* 8.1 Hz, 1H), 5.99 (s, 1H), 5.44 (s, 0H), 5.49 - 5.38 (m, 1H), 3.59 (dd, *J* = 15.9, 9.4 Hz, 1H), 3.18 (ddt, *J* = 15.9, 8.0, 1.0 Hz, 1H). ¹³C NMR (126 MHz, acetone) δ 155.05, 138.78, 128.91, 125.31, 125.28, 124.21, 121.27, 121.24, 121.21, 119.55, 118.01, 117.99, 116.95, 116.92, 112.18, 107.13, 56.18, 37.03. HPLC-MS (15-95, gt: 10 min) t_{R}: 6.96 min, *m*/*z* = 371 [MH]⁺, purity = 99%.

5-Methoxy-3-(5-methoxy-1-(prop-2-yn-1-yl)indolin-2-yl)-1-(prop-2-yn-1-yl)-1*H*-indole **(8).** White solid (60% yield). ¹H NMR (500 MHz, acetone-*d*₆) δ 7.43 (dd, *J* = 8.9, 0.6 Hz, 2H), 7.23 (dd, *J* = 2.5, 0.5 Hz, 1H), 6.88 (ddd, *J* = 8.9, 2.5, 0.5 Hz, 1H), 6.80 - 6.78 (m, 1H), 6.71 (ddq, *J* = 8.4, 2.6, 0.5 Hz, 1H), 6.63 (d, *J* = 8.4 Hz, 1H), 5.04 (s, 1H), 5.04 (s, 1H), 4.86 (dd, *J* = 11.9, 8.4 Hz, 1H), 4.07 (dd, *J* = 17.9, 2.4 Hz, 1H), 3.74 (s, 3H), 3.74 (s, 3H), 3.57 (dd, *J* = 18.0, 2.4 Hz, 1H), 3.26 (ddd, *J* = 15.3, 8.5, 0.8 Hz, 1H), 3.16 (ddt, *J* = 15.4, 12.0, 1.2 Hz, 1H), 2.94 (t, *J* = 2.8 Hz, 1H), 2.57 (t, *J* = 2.5 Hz, 1H). ¹³C NMR (126 MHz, acetone-*d*₆) δ 188.77, 174.69, 161.34, 127.22, 111.78, 111.70, 111.25, 110.69, 109.04, 101.93, 98.75, 73.73, 72.76, 61.39, 60.72, 55.01, 45.86, 36.89, 35.32. HPLC-MS (15-95, gt: 10 min) t_{R}: 4.23 min, *m*/*z* = 371 [MH]⁺, purity = 98%.

### Example 1.2. Evaluation of physicochemical properties

The Oxygen Radical Absorbance Capacity Assay (ORAC) was performed as described [Herrera-Arozamena, Clara et al. "Resveratrol-Based MTDLs to Stimulate Defensive and Regenerative Pathways and Block Early Events in Neurodegenerative Cascades." Journal of medicinal chemistry vol. 65,6 (2022): 4727-4751. doi:10.1021/acs.jmedchem.1c01883]. The results are expressed as trolox equiv. in a comparative scale that shows if a compound is a better (ORAC > 1.0) or a worse ROS scavenger (ORAC < 1.0) than trolox, the aromatic part of vitamin E and responsible for antioxidant properties.

The *in vitro* blood-brain barrier permeation assay (PAMPA-BBB) was used for predicting the brain penetration of compounds, following a described method [Herrera-Arozamena, Clara et al. "Resveratrol-Based MTDLs to Stimulate Defensive and Regenerative Pathways and Block Early Events in Neurodegenerative Cascades." Journal of medicinal chemistry vol. 65,6 (2022): 4727-4751. doi:10.1021/acs.jmedchem.1c01883]. The results are given as the mean ± SD. In each experiment, 11 quality control standards of known BBB permeability were included to validate the analysis set.

The inhibition of human lipoxygenase-5 (hLOX-5) was performed as described [Herrera-Arozamena, Clara et al. "Tuning melatonin receptor subtype selectivity in oxadiazolone-based analogues: Discovery of QR2 ligands and NRF2 activators with neurogenic properties." European journal of medicinal chemistry vol. 190 (2020): 112090. doi:10.1016/j.ejmech.2020.112090], Data are the mean ± SD of three independent experiments performed in triplicate.

### Example 1.3. In vitro evaluation of biological properties

Screening of NRF2 induction was quantified by luciferase activity in AREc32 cells as described in [Herrera-Arozamena, Clara et al. "Tuning melatonin receptor subtype selectivity in oxadiazolone-based analogues: Discovery of QR2 ligands and NRF2 activators with neurogenic properties." European journal of medicinal chemistry vol. 190 (2020): 112090. doi:10.1016/j.ejmech.2020.112090].

Screening for anti-inflammatory properties in primary rat glial cultures was performed according to the protocol described previously [Gameiro, Isabel et al. "Discovery of the first dual GSK3β inhibitor/Nrf2 inducer. A new multitarget therapeutic strategy for Alzheimer's disease." Scientific reports vol. 7 45701. 31 Mar. 2017, doi:10.1038/srep45701]. All compounds were evaluated at the single concentration of 3 µM. Neuroinflammation was quantified as the value of released nitrates, measured by the Griess method, normalized by the number of viable cells measured by the MTT method [Mosmann, T. "Rapid colorimetric assay for cellular growth and survival: application to proliferation and cytotoxicity assays." Journal of immunological methods vol. 65, 1-2 (1983): 55-63. doi:10.1016/0022-1759(83)90303-4]. LPS alone was considered 100 % inflammation.

Nitric oxide (NO) detection in the mouse microglial cell line BV2 was evaluated by using the fluorescent probe 4-amino-5-methylamino-2',7'-difluorofluorescein diacetate (DAF) with excitation and emission wavelengths of 495 nm of 515 nm, respectively according to the manufacturer's instructions. Fluorescence results were divided by cell viability (measured as MTT reduction) and normalized to basal condition. Results were normalized respect to basal condition.

Immunofluorescence labelling in *in vitro* and *in vivo* samples was performed as described in [Luengo, Enrique et al. "Implication of type 4 NADPH oxidase (NOX4) in tauopathy." Redox biology vol. 49 (2022): 102210. doi:10.1016/j.redox.2021.102210 ].

Tau seeding assay in HEK-293 was evaluated following a previously described protocol [Holmes et al. "Proteopathic tau seeding predicts tauopathy in vivo." Proceedings of the National Academy of Sciences 2014, 111, E4376-E4385].

### Example 1.4. In vivo experiments

The LPS *in vivo* model was induced, sickness behaviour evaluated (locomotion test and novel object recognition test) and cytokines measured by qPCR as described by [Fernández-Mendivil, Cristina et al. "Protective role of microglial HO-1 blockade in aging: Implication of iron metabolism." Redox biology vol. 38 (2021): 101789. doi:10.1016/j.redox.2020.101789]. The panel of mouse cytokines, chemokines, and growth factors were measured by Luminex xMAP technology for multiplexed quantification at Evetechnologies (https://www.evetechnologies.com/)

*In vivo* hTau301L model was induced by stereotaxic injection of AAV-hTauP301L (AAV-hTau) particles as described in [Luengo, Enrique et al. "Implication of type 4 NADPH oxidase (NOX4) in tauopathy." Redox biology vol. 49 (2022): 102210. doi:10.1016/j.redox.2021.102210 ]. Control animals were injected with AAV-GFP. Evaluation of hyperphosphorylated tau in sarkosyl soluble and insoluble fractions; microgliosis; AT8 and AT180 by immunofluorescence; and cognitive test were performed as described previously [Luengo, Enrique et al. "Implication of type 4 NADPH oxidase (NOX4) in tauopathy." Redox biology vol. 49 (2022): 102210. doi:10.1016/j.redox.2021.102210 ].

Oral administration of Compound 1 at different dosages (20 mg·kg⁻¹ and 50 mg·kg⁻¹) was administered embedded in gelatine. The compound was given in a mixture of 10 % gelatine from porcine skin and 10 % sucralose in water, heated in a microwave oven. Before Compound 1 addition, gelatine mixture was left at RT. Afterwards, the compound was added, evenly mixed and plated 1 mL·well⁻¹ in a P24 plate. Before AAV injection, mice were trained to eat the gelatines by giving them one gelatine (without compound) per day for 4 consecutive days. After AAV injection, mice were treated once a day with control or Compound 1 gelatines for 35 consecutive days. This approach was followed in adult (3-4 months old) and aged (16-18 months old) mice injected with AAV-GFP or AAV-hTauP301L.

### Example 1.5. Data and statistical analysis

Mice were randomly assigned to each treatment group. Data analysis was performed in a double-blinded manner. Unless otherwise stated, all data are presented as mean ± SEM. Statistics was performed using GraphPad Prism (v.8.0.2.). The number of biological replicates (n) is mentioned in the respective figure or graphs. In some cases, group sizes became unequal during the study due to biological loss, technical failure or presence of outliers that were excluded using predefined criteria. Data was tested for normality to determine whether to use a parametric or non-parametric test. Outliers were identified following ROUT method (Q= 1%). Unless otherwise mentioned, for grouped comparisons one-way ANOVA with Tukey's post-hoc test was used. Statistical differences were set as *p<0.05, **p<0.01 and ***p<0.005 or #p<0.05, ##p<0.01 and ###p<0.005.

### Example 2. Results

### Example 2.1. In vitro characterization of the compounds of the invention according to their anti-neuroinflammatory properties

Based on the results obtained **(Table 2),** Compound 1 was selected for further *in vitro* and *in vivo* studies as it was endowed with the best anti-neuroinflammatory activity.

**Table 2: Comparative in vitro results. Cell viability/toxicity: effect of the compound per se on cell viability of AREc332 cells measured with the MTT method. Anti-neuroinflammatory effect: % Reduction of nitrites in primary rat glial cultures. PAMPA-BBB: In vitro blood-brain barrier permeation assay, permeation rate Pₑ (10⁻⁶ cm.s⁻¹). NRF2, CD (µM): Concentration of compound that doubles de activation of NRF2. ORAC: Oxygen radical absorbance capacity. LOX-5: lipoxygenase-5.**

| Cell | Compd. ID Viability (CC₅₀ µM) | Anti-neuro inflammatory effect at 3 µM (%) | PAMPA-BBB *P*ₑ (10⁻⁶ cm.s⁻¹) | NRF2 CD (µM) | ORAC (trolox equiv.) | LOX5 IC₅₀, µM |
|---|---|---|---|---|---|---|
| 1 | >30 | 100 ± 6.6 | 12.9 ± 0.9 (cns+) | 0.56 ± 0.05 | 2.3 ± 0.3 | 3.08±0.29 |
| 2 | 3<CC₅₀<10 | 33.2 ± 11.8 | 8.7 ± 0.6 (cns+) | 0.329 ± 0.017 | 1.5 ± 0.2 | 0.42±0.03 |
| 3 | >30 | 24.4± 6.7 | 11.5 ± 0.8 (cns+) | 0.0713±0.031 | 1.9 ± 0.1 | 2.89±0.44 |
| 4 | 10<CC₅₀<30 | 29.6 ± 9.4 | 5.2 ± 0.4 (cns+) | 2.04 ± 0.20 | 1.6 ± 0.2 | 4.57±0.25 |
| 5 | >30 | 30 ± 10 | 10.9 ± 0.7 (cns+) | 2.57 ± 0.68 | 0.5 ± 0.1 | >100 |
| 6 | >30 | 45 ± 8.7 | 18.5 ± 0.8 (cns+) | 3.27 ± 0.25 | 1.0 ± 0.1 | >100 |

| Compd. ID | Cell Viability (CC₅₀ µM) | Anti-neuro inflammatory effect at 3 µM (%) | - BBB *P*ₑ (10⁻⁶ cm.s⁻¹) | NRF2 CD (µM) | ORAC (trolox equiv.) | LOX5 IC₅₀, µM |
|---|---|---|---|---|---|---|
| 7 | 10<CC₅₀<30 | 45 ± 7.1 | 18.1 ± 0.9 (cns+) | >30 | 1.3 ± 0.1 | 6.63±0.21 |
| 8 | >30 | 37 ± 11 | 8.3 ± 0.3 (cns+) | 10.7 ± 1.3 | 1.3 ± 0.1 | 0.53±0.02 |

**Example 2.2. *In vitro* treatment with Compound 1 has anti-inflammatory effects in microglial derived cells.** Compound 1 showed a concentration-dependent reduction of nitric oxide (NO) in BV2 cells exposed to LPS; at 10 µM, NO production was reduced to basal levels **(****Figure 1A****).** Compound also reduced the proinflammatory cytokine TNF-α mRNA in a concentration-dependent manner at 10 and 30 µM.

### Example 2.3. Compound 1 presents anti-neuroinflammatory properties in vivo

**Example 2.3.1. Treatment with Compound 1 reduces LPS-induced cytokine expression and release in mouse brain.** Mice were treated i.p. with a daily dose of 20 mg·kg⁻¹ of Compound 1 for 4 consecutive days. On the last day, LPS (0.5 mg·kg⁻¹) was injected i.p. one h after the compound. Thereafter, behavioural, and biochemical outcomes were analysed at different time points.

LPS produced an increase of several cytokines in the brain after its administration, Compound 1 reduced IL-1β and TNFα mRNAs 4 h and 24 h after LPS administration **(****Figures 2A, B****),** being the peak of both cytokines higher at 4 h post LPS. Also, cytokines like IL-6 and IL-7 **(****Figure 2** **D, E),** measured by multiplex ELISA, were significantly increased by LPS, especially after 4 h post LPS administration; mice treated with Compound 1 showed a marked reduction of these cytokines at 4 h, while their levels were restored to basal conditions after 24 h. A similar picture was observed for granulocyte-colony stimulating factor (G-CSF); LPS increased its brain levels while this increase was significantly reduced in animals treated with Compound 1.

Certain chemokines implicated in the extravasation of immune cells such as IP10, MCP-1, KC, MIP2 and RANTES were also evaluated. These were overexpressed upon LPS treatment at 4 and 24 h after LPS inoculation and decreased significantly with Compound 1 treatment except for MCP-1 at 24 h **(****Figures 2C****, F-J).** Additional cytokines were studied at both timelines **(****Figure 3****),** most of them were increased upon the inflammatory condition and decreased with compound treatment.

**Example 2.3.2. Treatment with Compound 1 reduces LPS-induced microgliosis in mouse brain.** Microglial cells constitute the immune cells of our brain; therefore, they play a key role in regulating neuroinflammation. Microgliosis was evaluated using Iba-1 as a microglial marker in immunofluorescence experiments 4 h and 24 h after the inflammatory stimuli (LPS). The mean fluorescence intensity of Iba-1 per cell in mice treated with LPS, mainly 4 h after LPS, was almost doubled respect saline condition; this increase was restored to saline levels in mice treated with Compound 1 both at 4 and 24 h **(****Figure 4****).**

**Example 2.3.3. Treatment with Compound 1 reduces LPS-induced sickness behaviour.** Peripheral administration of LPS produces neuroinflammation and induces a "sickness behaviour" in mice that is characterized by body weight loss, reduction of the locomotor activity and a cognitive decline. Mice treated with Compound 1 showed less reduction of the body weight loss compared to LPS-vehicle treated animals **(****Figure 5A****).** Locomotion was also improved in mice treated with Compound 1 measured as line crossings of an imaginary grid 4 and 8 h after LPS exposure **(****Figure 5B****);** these values were similar to saline animals. Interestingly, mice treated with Compound 1 did not show cognitive decline compared to vehicle-LPS treated animals **(****Figure 5C and D****).** Taken together, Compound 1, significantly reduced the sickness behaviour that accompanies the neuroinflammatory response induced by LPS treatment.

**Example 2.4. Compound 1 reduces levels of pathological hyperphosphorylated tau in primary cultures of neurons expressing human tau protein with P301L mutation.** At day 10 after culture, neurons were treated with PBS or AAV-hTauP301L, and thereafter compound 1 (30 µM) was added every other day for 8 days. At the end of the experiment (day 18), neurons were fixed in 2 % paraformaldehyde (Sigma-Aldrich) for 15 minutes. Thereafter, hyperphosphorylated tau levels were evaluated by immunofluorescence using AT8 antibody **(****Figure 6A****).** Compound 1 reduced AT8 intensity levels as well as dimethylfumarate (DMF), our positive control **(****Figure 6B****).**

In a second set of experiments, HEK-293 FRET biosensor containing tau repeating domains was used to determine if Compound 1 could reduce tau aggregates. For this, cells were treated with Tg4510 mouse brain homogenate as an aggregant stimulus and co-incubated with increasing concentrations of the compound. Compound 1 was able to reduce tau seeding ability following a concentration dependent effect **(****Figure 6C****).**

**Example 2.5. Compound 1 ameliorates hTauP301L induced FTD and cognitive decline in adult (3-4 months old) mice.** FTD was generated in adult male and female C57BL/6J mice (3-4 months old) by bihippocampal injection of AAV-hTau particles containing the P301L mutation. Mice were treated orally once a day with a single dose of 20 or 50 mg/kg of Compound 1 formulated in gelatines the day after stereotaxic delivery of AAVs (GFP or TauP301L, to controls and FTD, respectively). After 35 days treatment, hyperphosphorylated tau levels were evaluated by immunofluorescence and by WB in soluble and insoluble in sarkosyl fractions. Also, behavioural tests were conducted to study the cognition status of the mice.

Hyperphosphorylated tau levels measured with the antibodies AT8 (p-Ser202/p-Thr205) and AT180 (p-Thr231) were analysed by immunofluorescence in fixed postmortem brain slices of treated mice **(****Figure 7A****).** Compound 1 treatment significantly reduced AT8 and AT180 fluorescence in all areas of the hippocampus analysed (DG, CA1 and CA3) **(****Figure 7B and C****).**

After 35 days of treatment, monomeric and oligomeric forms of tau, soluble and insoluble in sarkosyl, were analysed by western blot. Hyperphosphorylated tau levels were studied with AT8 (p-Ser202/p-Thr205) and AT180 (p-Thr231) antibodies **(****Figure 8****).** Animals with FTD treated with Compound 1 showed significant reduction of insoluble monomers and a tendency to reduce insoluble oligomers of AT180 **(****Figure 8F****).**

Spatial memory was measured using the T-maze **(****Figure 9A****)** and the Object localization tests (OLT; **Figure 9B****).** T maze determines short term spatial memory and the exploratory behavior of mice. h-TauP301L mice showed a significant 20 % reduction in the percentage of success. Mice treated for 35 days with 20 mg/kg or 50 mg/kg showed a dose-dependent improvement; at 50 mg/kg total recovery was achieved **(****Figure 9C****).** In the OLT a similar pattern was observed; Compound 1 showed a dose-dependent improvement and total recovery was achieved with the dose of 50 mg/kg **(****Figure 9D****).**

**Example 2.6. Compound 1 ameliorates hTauP301L induced FTD and cognitive decline in aged (16-18 months old) mouse.** To include the aging effect in the FTD model, the previous experiments were replicated in male and female 16-18 months old C57/BL6J mice.

Similar results to those obtained in adult subjects were obtained. 35 Days of treatment with a single oral dose of Compound 1 at 50 mg/kg reduced hyperphosphorylated tau levels induced by hTauP301L, measured by AT8 and AT180 antibodies in DG, CA3 and CA1 regions of the hippocampus **(****Figure 10A-C****).** Furthermore, spatial memory loss caused by AAV-hTauP301 was prevented in mice treated with compound 1 as indicated in the T-maze **(****Figure 10D and F****)** and OLT **(****Figure 10E and G****)** behavioural tests.

Taken together, Compound 1 at 50 mg/kg administered orally once a day for 35 days caused significant reduction of hyperphosphorylated tau and prevented cognitive decline induced by hTauP301L both in young and aged subjects.

## Claims

1. Compound **characterized by** comprising the *Formula I,* or salts or derivatives thereof, 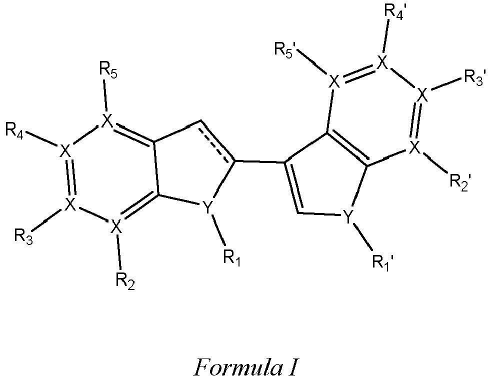 wherein,
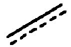 represents a double or single bond,
**X** is either C or N,
**Y** is N, O or S,
**R₁** and **R₁'** are independently absent, H, C₁₋₄ alkyl, or an acyl group CO**R₆;** wherein
**R₆** is H or C₁₋₄ alkyl; and wherein the C₁₋₄ alkyl comprises cycloalkyl, branched alkyl, alkenyl or alkynyl, optionally substituted with 1-3 halogen atoms,
**R₂, R₃**, **R₄, R₅**, **R₂' R₃', R₄'** and **R₅'** are independently absent, H, carbonitrile, C₁₋₄ alkyl, a halogen, aryl, heteroaryl, an alkoxy group O**R₇**, or an amino group N**R**₈**R**₉; wherein **R**₇, **R**₈ and **R**₉ are H or C₁₋₄ alkyl; and wherein C₁₋₄ alkyl comprises cycloalkyl, branched alkyl, alkenyl or alkynyl, optionally substituted with 1-3 halogen atoms;
for use in the treatment of frontotemporal dementia or frontotemporal lobar dementia.

2. Compound, for use, according to claim 1, **characterized in that** the compound comprises the *Formula II,* or salts or derivatives thereof, 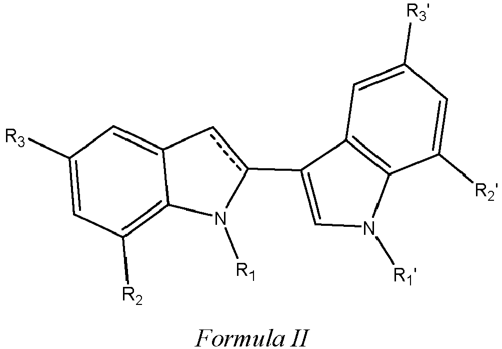 wherein,
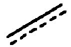 represents a double or single bond,
**R₁** and **R₁'** are independently H or C₁₋₄ alkyl, wherein the C₁₋₄ alkyl comprises cycloalkyl, branched alkyl, alkenyl or alkynyl, optionally substituted with 1-3 halogen atoms,
**R₂** and **R₂'** are independently H or an alkoxy group O**R₇**, where **R₇** is H or C₁₋₄ alkyl, wherein the C₁₋₄ alkyl comprises cycloalkyl, branched alkyl, alkenyl or alkynyl, optionally substituted with 1-3 halogen atoms,
**R₃** and **R₃'** are independently H or an alkoxy group O**R₇**, where **R₇** is H, C₁₋₄ alkyl, wherein the C₁₋₄ alkyl comprises cycloalkyl, branched alkyl, alkenyl or alkynyl optionally substituted with 1-3 halogen atoms, a halogen atom or C₁₋₄ alkyl comprising cycloalkyl, optionally substituted with 1-3 halogen atoms.

3. Compound, for use, according to any of the previous claims, wherein **R₁** and **R₁'** are both H or prop-2-ynyl, **R₂** and **R₂'** are both H or methoxy and/or **R₃** and **R₃'** are both H, methoxy, chloro or trifluoromethyl.

4. Compound, for use, according to any of the previous claims, wherein **R₁** and **R₁'** are both H, **R₂** and **R₂'** are both H or methoxy and **R₃** and **R₃'** are both H, methoxy, chloro or trifluoromethyl.

5. Compound, for use, according to any of the previous claims, wherein **R₂** and **R₂'** are both H or methoxy and **R₃** and **R₃'** are both H.

6. Compound, for use, according to any of the previous claims, wherein **R₃** and **R₃'** are both H, methoxy, chloro or trifluoromethyl and **R₂** and **R₂'** are both H.

7. Compound, for use, according to any of the previous claims, wherein **R₁** and **R₁'** are both prop-2-ynyl, **R₃** and **R₃'** are both methoxy and **R₂** and **R₂'** are both H.

8. Compound for use, according to any of the previous claims, wherein the compound of *Formula I* or *Formula II* is selected from the group comprising:
5-Methoxy-3-(5-methoxyindolin-2-yl)-1*H*-indole **(1)**
5,5'-Dimethoxy-1*H*,1'*H*-2,3'-biindole **(2)**
3-(Indolin-2-yl)-1*H*-indole **(3)**
5-Chloro-3-(5-chloroindolin-2-yl)-1*H*-indole **(4)**
5,5'-Dichloro-1*H*,1'*H*-2,3'-biindole **(5)**
7-Methoxy-3-(7-methoxyindolin-2-yl)-1*H*-indole **(6)**
5-(Trifluoromethyl)-3-(5-(trifluoromethyl)indolin-2-yl)-1*H*-indole **(7)**
5-Methoxy-3-(5-methoxy-1-(prop-2-yn-1-yl)indolin-2-yl)-1-(prop-2-yn-1-yl)-1*H-*indole **(8)**

9. Compound for use, according to any of the previous claims, wherein the compound of *Formula I or Formula II* is:
5-Methoxy-3-(5-methoxyindolin-2-yl)-1*H*-indole **(1)**

10. Compound for use, according to any of the previous claims, wherein the frontotemporal dementia or frontotemporal lobar dementia is **characterized by** the presence of mutations in the MAPT gene.

11. Compound for use, according to any of the previous claims, wherein the frontotemporal dementia or frontotemporal lobar dementia is selected from the list comprising: Pick's disease, corticobasal degeneration, progressive supranuclear palsy, argyrophilic grain disease, multiple system tauopathy with dementia, neurofibrillary tangle predominant dementia and/or white matter tauopathy with globular glial inclusions.

12. Compound for use, according to any of the previous claims, wherein the frontotemporal dementia or frontotemporal lobar dementia is **characterized by** the presence of mutation P301L in the MAPT gene.

13. Compound for use, according to any of the previous claims, wherein the compound is administered *via* parenteral or enteral route, preferably via sublingual, buccal, oral and rectal routes.

14. Compound for use, according to any of the previous claims, wherein the compound is administered *via* enteral or parenteral route at a concentration ranging from 20 to 100 mg/kg.

15. Pharmaceutical composition comprising the *Formula I or Formula II,* according to any of the claims 1 to 13, or salts or derivatives thereof, and optionally pharmaceutically acceptable excipients and/or carriers, for use in the treatment of frontotemporal dementia or frontotemporal lobar dementia.
